(19) Europäisches Patentamt
European Patent Office
Office européen des brevets

(11) EP 2 786 731 B1

## (12) EUROPEAN PATENT SPECIFICATION

(45) Date of publication and mention
of the grant of the patent:
**25.12.2019 Bulletin 2019/52**

(21) Application number: **13741158.3**

(22) Date of filing: **25.01.2013**

(51) Int Cl.:
*A61F 13/15* *(2006.01)*    *A61F 13/49* *(2006.01)*
*A61F 13/53* *(2006.01)*    *A61F 13/532* *(2006.01)*
*A61F 13/533* *(2006.01)*    *A61F 13/535* *(2006.01)*

(86) International application number:
**PCT/JP2013/051634**

(87) International publication number:
**WO 2013/111871 (01.08.2013 Gazette 2013/31)**

(54) **DISPOSABLE ARTICLE OF CLOTHING**

EINWEG-BEKLEIDUNGSARTIKEL

ARTICLE JETABLE À PORTER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.01.2012 JP 2012015271**

(43) Date of publication of application:
**08.10.2014 Bulletin 2014/41**

(73) Proprietor: **Unicharm Corporation
Ehime 799-0111 (JP)**

(72) Inventors:
• **ARAYAMA, Takaya
Kanonji-shi
Kagawa 769-1602 (JP)**

• **MUKAI, Hirotomo
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
**EP-A2- 1 275 358       WO-A1-2004/000188
WO-A1-2012/005249       WO-A1-2012/067217
WO-A2-03/026545         JP-A- H08 280 738
JP-A- 2002 320 641      JP-A- 2006 055 360
JP-A- 2009 125 199      JP-A- 2009 160 035**

EP 2 786 731 B1

**Description**

[Technical Field]

The present invention relates to a disposable diaper having an absorbent polymer that absorbs body fluid.

[Background Art]

In recent years, high absorption performance is required in a disposable wearing article such as a pants-type diaper, and various types of disposable wearing articles having an absorbent polymer that absorbs body fluid have been provided. For example, Patent Literature 1 describes a disposable wearing article having a super absorbent material as an absorbent polymer. The disposable wearing article has a backsheet and an absorbent structure arranged on the backsheet. The absorbent structure includes an absorbent layer having a front end and a rear end, and a serge layer arranged on the absorbent layer.

An absorbent panel is laminated at the front end of an absorbent layer, and the absorbent layer and the absorbent panel are laminated at the front end of the absorbent structure, thus forming a thick front absorbent region. The absorbent layer and the absorbent panel included in the front absorbent region contain the super absorbent material described. According to the disposable wearing article configured as described above, it is possible to ensure absorption performance by arranging a large amount of the super absorbent material in the front absorbent region that is in contact with a front side of a wearer, and it is thus possible to absorb body fluid excreted by the wearer in the front absorbent region.

[Citation List]

[Patent Literature]

[PTL 1] Japanese Unexamined Patent Application Publication No. H08-66426 (Fig. 3, Fig. 5, Paragraphs 0011, 0015, 0016, etc.)

[Summary of Invention]

However, the inventor(s) has/have recognized that, in the above-described disposable wearing article ,Since a large amount of the super absorbent material is arranged in the front absorbent region, the body fluid can be absorbed concentratedly in the front absorbent region. However, the body fluid excreted from a wearer is excreted in a large amount in an urination region substantially in the center in a front-back direction of the absorbent structure.

The front absorbent region is separated from the urination region where a large amount of the body fluid is excreted, because of which the body fluid may not be absorbed effectively and the absorption capacity of the front absorbent region may not be exhibited sufficiently.

Further, the absorbent structure is substantially rectangular in a plan view, and has the same length in the widthwise direction of an urination region arranged between both legs of the wearer and of a front absorbent region in which a large amount of the super absorbent material is arranged. However, the urination region positioned between the both legs of the wearer is compressed by the both legs, wrinkles along the front-back direction might be formed in the urination region. When the wrinkles formed in the urination region are propagated to the front absorbent region, wrinkles are formed also in the front absorbent region, so that the absorption capability of the super absorbent material arranged in the front absorbent region cannot be exhibited sufficiently in some cases.

Thus, an object of the present invention is to provide a disposable wearing article with which it is possible to improve an absorption capacity of an entire absorbent article by making effective use of the absorption capacity by an absorbent polymer.

[Solution to Problem]

A disposable diaper according to claim 1.

[Advantageous Effects of Invention]

A disposable diaper according to the present invention, since a deformation restraining unit that restrains deformation of the urination region from being propagated to an absorbent sheet or a first region is formed, even when the urination region that is in contact with a urination portion of a wearer is deformed by being interposed between the both legs and the like, it becomes less likely that wrinkles are generated in the absorbent polymer arranged regions or the first region. By restraining generation of wrinkles, swelling of the absorbent polymer is less likely to be inhibited, thus making it possible to improve absorption efficiency and secure an absorption area to reduce leakage. Absorption performance on the basis of an absorption area and absorption efficiency is improved, thereby making it possible to make effective use of the absorption capacity by an absorbent polymer to improve an absorption capacity of an entire absorbent article. In particular, even after a body fluid is absorbed, a good feeling of wearing can be obtained.

[Brief Description of Drawings]

[Fig. 1] Fig. 1 is a schematic perspective view of a disposable diaper according to a first embodiment.
[Fig. 2] Fig. 2 is a developed plan view of the disposable diaper according to the first embodiment.
[Fig. 3] Fig. 3 is a cross-sectional view in the widthwise direction of the disposable diaper taken along the X1-X'1 line shown in Fig. 2.
[Fig. 4] Fig. 4 is a cross-sectional view in the lengthwise direction of the disposable diaper taken along the X2-X'2 line shown in Fig. 2.
[Fig. 5] Fig. 5 is a cross-sectional view in the widthwise direction of the disposable diaper taken along the X3-X'3 line shown in Fig. 2.
[Fig. 6] Fig. 6 is a plan view of an absorber according to the first embodiment.
[Fig. 7] Fig. 7 is a cross-sectional view along the X1-X'1 line that schematically illustrates a deformed state of the disposable diaper according to the first embodiment.
[Fig. 8] Fig. 8 is a developed plan view of an absorber of a disposable diaper according to a second embodiment not part of the invention.
[Fig. 9] Fig. 9 is a developed plan view of an absorber of a disposable diaper according to a third embodiment not part of the invention.
[Fig. 10] Fig. 10 is a cross-sectional view in the widthwise direction of the disposable diaper taken along the X4-X'4 line shown in Fig. 9.
[Fig. 11] Fig. 11 is a cross-sectional view in the widthwise direction of the disposable diaper taken along the X5-X'5 line shown in Fig. 9.
[Fig. 12] Fig. 12 is a developed plan view of an absorber of a disposable diaper according to a fourth embodiment not part of the invention.
[Fig. 13] Fig. 13 is a cross-sectional view in the widthwise direction of the disposable diaper taken along the X6-X'6 line shown in Fig. 11.
[Fig. 14] Fig. 14 is a developed plan view of an absorber of a disposable diaper according to a fifth embodiment not part of the invention.

<First embodiment>

Next, a disposable diaper 1 according to the first embodiment is explained with reference to drawings. Note that in the description of the following drawings, the same or similar symbols are added to the same or similar portions. However, it must be taken into consideration that the drawings are schematic representations and are not drawn to scale unless otherwise specified. Moreover, the drawings do not necessarily reflect the actual dimensional relationships and ratios of components. Therefore, the specific dimensions must be determined in view of the following explanation. Furthermore, relations or ratios among such dimensions may be different from one drawing to another.

The disposable wearing article according to the first embodiment is characterized in that an absorbent sheet having an absorbent polymer that absorbs body fluid is provided in a front waistline region S1 as a ventral region.

(1) Overall schematic configuration of the disposable wearing article

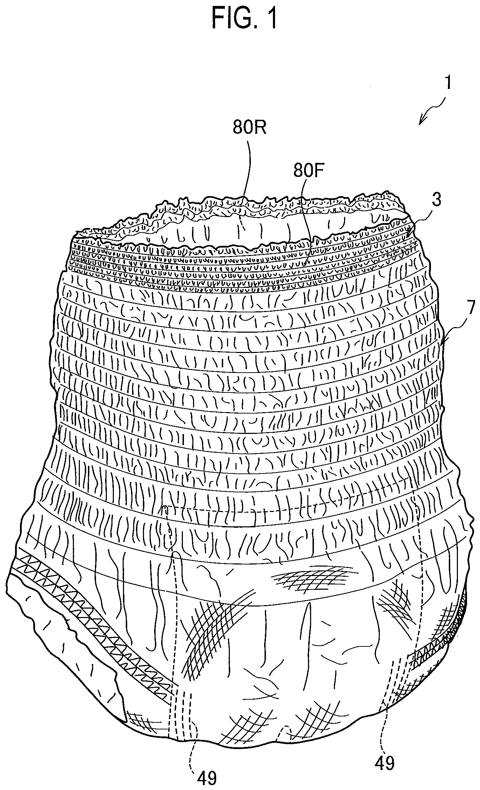

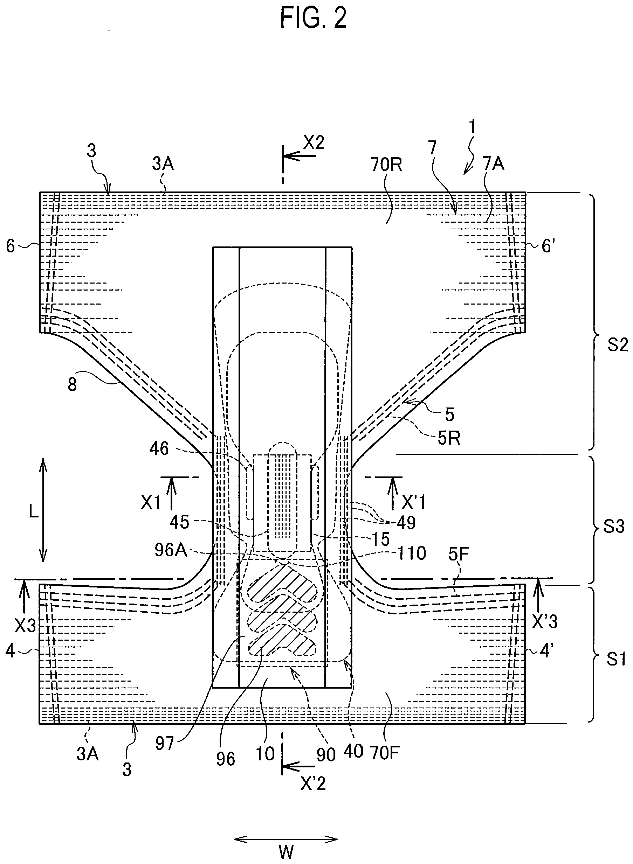

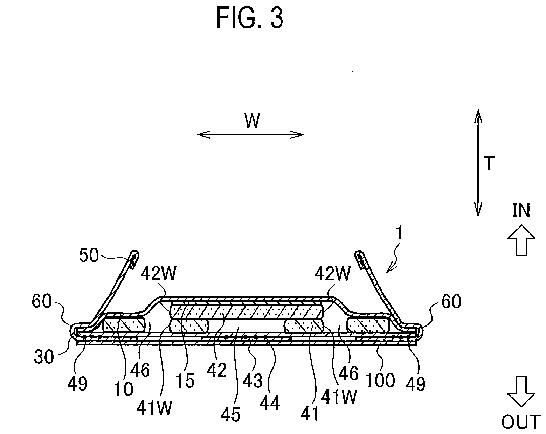

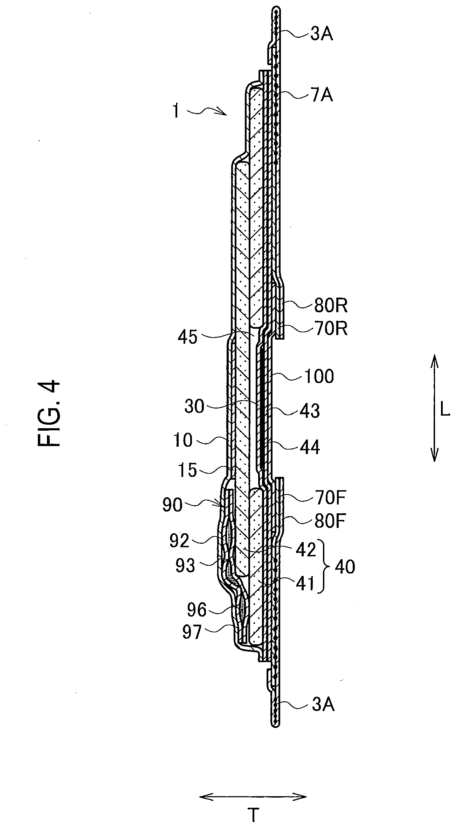

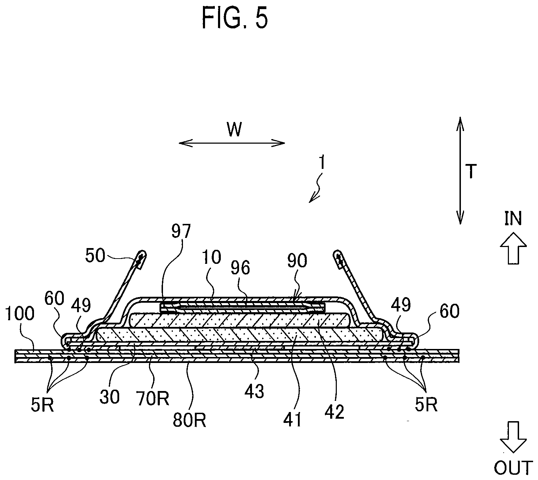

Fig. 1 is a schematic perspective view of the disposable diaper 1 configuring the disposable wearing article in the present embodiment. Fig. 2 is a developed plan view of the disposable diaper 1 according to the present embodiment. Fig. 3 is a cross-sectional view in the widthwise direction of the disposable diaper taken along the X1-X'1 line shown in Fig. 2. Fig. 4 is a cross-sectional view in the lengthwise direction of the disposable diaper taken along the X2-X'2 line shown in Fig. 2. Fig. 5 is a cross-sectional view in the widthwise direction of the disposable diaper taken along the X3-X'3 line shown in Fig. 2. The disposable diaper 1 is a pants-type disposable diaper.

As shown in Fig. 2, the disposable diaper 1 has, in the front-back direction of the disposable diaper 1, the front waistline region S1 corresponding to the front waistline of a wearer, a rear waistline region S2 corresponding to the rear waistline of the wearer, and a urination region S3 corresponding to the crotch of the wearer, the urination region S3 being a region in contact with the urination portion of the wearer. The urination region S3 is a region which is closer to the front and includes the center of the absorber in the front-back direction. The rear waistline region S2 is a region that is to the rear of the center of the absorber in the front-back direction, and is arranged to the rear of the urination region S3, thus serving as the buttocks region, which is the region in contact with the buttocks of the wearer. The front waistline region S1 is a region that is to the front of the center of the absorber in the front-back direction, and is arranged to the front of the urination region S3, thus serving as the ventral region, which is the region in contact with the ventral of the wearer.

A front waistband edge 4, positioned on the outside of the front waistline region S1 in a widthwise direction W of the disposable diaper 1, is joined to a rear waistband edge 6, positioned on the outside of the rear waistline region S2 in the widthwise direction W; also, a front waistband edge 4' is joined to a rear waistband edge 6', whereby the disposable diaper 1 is formed into the pants-type.

The disposable diaper 1 includes a topsheet 10, an absorber 40, a sidesheet 60, a foreside exterior topsheet 70F, a rearside exterior topsheet 70R, an exterior center sheet 100, a foreside exterior backsheet 80F, a rearside exterior backsheet 80R, and the like, each of these elements being joined to each other by an adhesive, thermal fusion bonding, or the like.

The foreside exterior topsheet 70F, the rearside exterior topsheet 70R, the foreside exterior backsheet 80F, the rearside exterior backsheet 80R, and the exterior center sheet 100 are sheets which constitute the exterior portion of the disposable diaper 1. The absorber 40 including cotton-like pulp and highly polymerized water absorbent polymer is provided on the inner side (skin contact surface side) of the foreside exterior topsheet 70F, the rearside exterior topsheet 70R, and the exterior center sheet 100.

The topsheet 10 is a sheet that forms the skin contact surface that may be in direct contact with the skin of the wearer. The topsheet 10 is formed of a liquid-permeable sheet, such as a hydrophilic nonwoven cloth and woven cloth, a perforated plastic film, or a perforated hydrophobic nonwoven cloth. The topsheet 10 according to the present embodiment is formed of a hydrophilic spun bond nonwoven cloth, having a basis weight of 23 g/m$^2$, made of polypropylene.

A second sheet 15 is joined with the non-skin contact surface side of the topsheet 10. The second sheet 15 is arranged between the topsheet 10 and the absorber 40. Providing the second sheet 15 makes it possible to increase the speed at which the body fluid is absorbed, and makes it possible to restrain reverse flow of the body fluid after absorption.

The second sheet 15 is made of, for example, an air-through nonwoven cloth, a porous film, or the like. The second sheet 15 according to the present embodiment is formed of (hydrophilic) air-through nonwoven cloth having a basis weight of 30 g/m$^2$.

The absorber 40 is arranged between a composite sheet in which the topsheet 10 and the second sheet 15 are joined, and an absorber backside-covering sheet 30.

The absorber 40 has a front-back direction going from the front waistline region S1 toward the rear waistline region S2, the widthwise direction W orthogonal to the front-back direction, an inner direction IN going toward the wearer who wears the disposable diaper 1, and an outer direction OUT going toward an opposite side of the inner direction. The absorber 40 is formed of a mixed powder of ground pulp, highly absorbent polymer, and the like.

The absorber 40 includes a first layer 41 positioned at the side of the non-skin contact surface with the wearer and a second layer 42 overlapping with the first layer 41 and positioned at the side of the skin contact surface with the wearer. The first layer 41 of the absorber 40 has a central aperture 45 formed in the center in the widthwise direction W. A pair of side slits 46 is formed on both outer sides of the central aperture 45 in the widthwise direction. A more detailed description of the configuration of the absorber 40 shall be provided later. The central aperture functions as a curving unit described below, and also functions as a guide portion.

The absorber 40 is joined between a composite sheet in which the topsheet 10 and the second sheet 15 are joined and an elastic member-covering sheet 43 by a hot-melt adhesive. The hot-melt adhesive is coated on each of the composite sheet and the elastic member-covering sheet 43, for example, 5 g/m$^2$ and 8 g/m$^2$ of the hot-melt adhesive may be coated, respectively, by a spiral coating method.

An absorbent sheet 90 is arranged on the skin contact surface side of the absorber 40 in the front waistline region S1. The absorbent sheet 90 is arranged to the front of the second sheet 15. The absorbent sheet 90 is a composite sheet sandwiching an absorbent polymer, and holds body fluid in the front waistline region, thus restraining the body fluid held by the absorber from returning toward the wearer. A more detailed description of the configuration of the absorbent sheet shall be provided later.

The disposable diaper 1 has a central elastic member 44 that is arranged to overlap the central aperture 45 in a thickness direction T of the disposable diaper 1. With these elastic members, slits, and the like formed in the absorber 40, the absorber 40 is configured to bend when the disposable diaper 1 is worn. In the present embodiment, the central elastic member 44 and the central aperture 45 constitute a curving unit formed to enable the absorber to bend convexly toward the inner direction. Furthermore, the side slits 46 are formed to enable the absorber to bend convexly toward the outer direction.

4

The sidesheet 60 is provided on both ends in the widthwise direction of the absorber 40 so as to integrally surround the topsheet 10 and the absorber backside-covering sheet. The sidesheet 60 is formed of a sheet such as a liquid-impermeable nonwoven cloth. The sidesheet 60 according to the present embodiment is made of an SMS nonwoven cloth (spun bond-meltblown-spun bond nonwoven cloth), having a basis weight of 15 g/m$^2$, made of polypropylene.

Both sidesheets overlap with each other at the widthwise ends of the sidesheets 60. The portion where the sidesheets overlap with each other has a leakage-preventing elastic member 50 (see Fig. 3) in a state of extending out along the front-back direction. The sidesheet 60 and the leakage-preventing elastic member 50 constitute a leakage-preventing wall which prevents side leakage of bodily waste.

The leakage-preventing wall is provided along the front-back direction of the disposable diaper 1, at both ends in the widthwise direction of the absorber 40. A plurality of leakage-preventing elastic members 50 are provided along the front-back direction of the disposable diaper 1 in a state of expanding out in the front-back direction of the disposable diaper 1, between the sidesheets 60 having been folded together.

The leakage-preventing elastic member 50 according to the present embodiment uses spandex, extended and fixed in two, at a thickness of 780 dtex and an extension rate of 3.0 times, on each of the right and left sides. The sidesheet 60 is fixed to the non-skin contact surface side of the absorber backside-covering sheet, and is folded toward the side of the topsheet from the both ends of the absorber in the widthwise direction. The sidesheets 60 are fixed to the topsheet 10 having a plurality of lines of hot-melt adhesive in the weight of 0.1 g/m$^2$ coated by a bead coating method.

The exterior topsheet includes the foreside exterior topsheet 70F arranged in the front waistline region S1 and the rearside exterior topsheet 70R arranged in the rear waistline region S2. In the thickness direction T, the foreside exterior topsheet 70F is arranged between the foreside exterior backsheet 80F and the absorber 40. In the thickness direction T, the rearside exterior topsheet 70R is arranged between the rearside exterior backsheet 80R and the absorber 40. In the front-back direction, the exterior center sheet 100 is arranged between the foreside exterior topsheet 70F and the rearside exterior topsheet 70R.

The front end of the exterior center sheet 100 is joined with the rear end of the foreside exterior topsheet 70F, and the rear end of the exterior center sheet 100 is joined with the front end of the rearside exterior topsheet 70R. The exterior center sheet 100 is arranged straddling the foreside exterior topsheet 70F and the rearside exterior topsheet 70R. The exterior center sheet 100 is joined to the surface side of the exterior topsheet by a hot-melt adhesive continuously coated thereon with a slot coater.

The exterior center sheet 100 is configured by a nonwoven cloth or the like. The exterior topsheet according to the present embodiment is configured by an SMS nonwoven cloth, having a basis weight of 15 g/m$^2$, made of polypropylene. When worn, the exterior center sheet 100 is positioned further inward (toward the skin contact surface) than the exterior topsheet.

In the front waistline region S1 and the rear waistline region S2, the foreside exterior topsheet 70F and the rearside exterior topsheet 70R are formed to have a width in the widthwise direction W greater than that of the urination region S3. The foreside exterior topsheet 70F and the rearside exterior topsheet 70R may be formed by an air-through nonwoven cloth, a spun bond nonwoven cloth, an SMS nonwoven cloth, a water proof film, or the like. The exterior topsheet according to the present embodiment is configured by an SMS nonwoven cloth, having a basis weight of 15 g/m$^2$, made of polypropylene.

In the front waistline region S1, the foreside exterior backsheet 80F is provided closer to the non-skin contact surface than the foreside exterior topsheet 70F. In the rear waistline region S2, the rearside exterior backsheet 80R is provided closer to the non-skin contact surface than the rearside exterior topsheet 70R. One end of the foreside exterior backsheet 80F in the front-back direction (the rearside exterior backsheet 80R) is folded toward the skin contact surface and is provided so as to surround an end of the foreside exterior topsheet 70F (the rearside exterior topsheet 70R) in the front-back direction.

The foreside exterior backsheet 80F and the rearside exterior backsheet 80R may be formed of an air-through nonwoven cloth, a spun bond nonwoven cloth, an SMS nonwoven cloth, a water proof film, or the like. The foreside exterior backsheet 80F and the rearside exterior backsheet 80R according to the present embodiment are configured by a spun bond nonwoven cloth, having a basis weight of 17 g/m$^2$, made of polypropylene.

The absorber backside-covering sheet 30 is partially attached to the foreside exterior topsheet 70F, the rearside exterior topsheet 70R, and the exterior center sheet 100.

Waist gathers 3 and waistband gathers 7 are provided in the front waistline region S1 and the rear waistline region S2. The waist gathers 3 and the waistband gathers 7 have elongated waist elastic members 3A and waistband elastic members 7A of synthetic rubber or the like that are laid out to stretch along the widthwise direction W of the absorber 40. The waist elastic members 3A and the waistband elastic members 7A are joined between the foreside exterior topsheet 70F and the foreside exterior backsheet 80F, and also between the rearside exterior topsheet 70R and the rearside exterior backsheet 80R with an adhesive (for example, the hot-melt adhesive) in an extended state in the widthwise direction W of the disposable diaper 1.

The waist gathers 3 and the waistband gathers 7 continue from one front waistband edge 4 up to the other front waistband edge 4' positioned on the outside in the widthwise direction W of the disposable diaper 1 in the front waistline region S1, and from one rear waistband edge 6 up to the other front waistband edge 6' positioned on the outside in the widthwise direction W of the disposable diaper 1 in the rear waistline region S2.

Leg gathers 5 are formed to run along the leg portions of the wearer. The leg gathers are formed of a synthetic rubber or other elongated leg hole elastic member laid out so as to stretch. The leg hole elastic member is configured by a front leg hole elastic member 5F arranged in the front waistline region S1, and a rear leg hole elastic member 5R arranged in the rear waistline region S2. The leg gathers 5 are provided so as not to cross the absorber 40.

The leg hole elastic member is joined between the foreside exterior topsheet 70F and the foreside exterior backsheet 80F, and between the rearside exterior topsheet 70R and the rearside exterior backsheet 80R.

Arranging the waist gathers 3, the waistband gathers 7, and the leg gathers 5 makes it possible to hold the disposable wearing article at the waistline, and makes it possible to prevent the entire disposable wearing article from slipping down. Accordingly, even in a case where the body fluid is held by the absorbent sheet 90 (described later) and the front side of the absorber becomes heavy, it is still possible to prevent the absorber from slipping down and possible to restrain the creation of a gap between the absorber 40 and the body of the wearer.

A hip elastic member according to the present embodiment is extended and fixed in sixes at a thickness of 940 dtex and an extension rate of 3.5 times, in both the front waistline region S1 and the rear waistline region. Further, the waistband elastic member is extended and fixed at a thickness of 780 dtex and an extension rate of 3.0 times. Examples of means for fixing the hip elastic member and the waistband elastic member may include the hot-melt adhesive. In the present embodiment, the elastic member is directly coated with the hot-melt adhesive using a V-slot method.

The leg hole elastic member according to the present embodiment is extended and fixed in threes at a thickness of 780 dtex and an extension rate of 1.5 to 3.5 times. The leg hole elastic member is arranged in a state where each portion has a gradient of a magnitude.

The leg hole elastic member is fixed by a hot-melt adhesive pre-coated onto the exterior topsheet. The hot-melt adhesive is coated thereon using a spiral spray. The amount coated on the leg hole elastic member is 7 $g/m^2$. The position of the vicinity of the end of the exterior topsheet (a position about 5 mm from the end) where there is overlap with at least the leg hole elastic member is coated with the adhesive using a slot coater. Coating the adhesive in this manner makes it possible to prevent the leg hole elastic member from falling out from the end of the exterior topsheet.

When the adhesive is coated on with a spiral spray which is a non-contact type, there is concern that the adhesive in the vicinity of the end of the exterior topsheet will jut out, and that a manufacturing defect will occur. However, coating with a slot coater which is a contact type makes it possible to prevent the adhesive from jutting out. The amount coated on with the slot coater is 110 $g/m^2$.

The central elastic member 44 is provided along the front-back direction and is provided at a position having overlap with the central aperture 45 in the thickness direction T of the disposable diaper 1. The central elastic member 44 is formed so as to be convex in the inner direction IN, i.e., so as to overlap onto the absorber 40 along the front-back direction, such that the absorber 40 bends convexly toward the wearer. Because the central elastic member 44 is arranged to overlap the central aperture 45, the central aperture 45 can be flexed upward (at the side of the wearer) more stably. The central elastic member 44 is arranged in an extended state along the front-back direction at the center in the widthwise direction of an absorbent article. The central elastic member 44 is arranged at the urination region S3.

The central elastic member 44 is provided in an extended state between the elastic member-covering sheet 43 and the absorber backside-covering sheet 30. The central elastic member 44 is arranged at an extension rate of 1.4 to 3.0 times. The central elastic member according to the present embodiment has a central elastic member positioned in the center in the widthwise direction, and an auxiliary central elastic member positioned on the outside in the widthwise direction of the central elastic member.

The central elastic members are arranged at the extension rate of 1.4 to 3.0 times. The central elastic member according to the present embodiment is extended and fixed in three at a thickness of 620 dtex and an extension rate of 2.5 times. The central elastic members have an interval of 5 mm there between and all have an adhesion length of 120 mm. The central auxiliary elastic member is arranged with less stress than the central elastic member, at a rate of 1.2 to 2.5 times. The central auxiliary elastic member according to the present embodiment is extended and fixed one by one at a thickness of 620 dtex and an extension rate of 1.8 times. The central auxiliary elastic member is arranged, at 5-mm intervals, in the same position, in the front-back direction, as the central elastic member, with a 5-mm interval, in the widthwise direction, from the both ends of the central elastic member.

The central auxiliary elastic member is arranged in order to alleviate convex-shape deformation of the absorber 40 at the central part in the widthwise direction of the urination region S3 at the time of wearing. By providing the central auxiliary elastic member, adhesion of the absorber 40 to the skin can be improved.

The central elastic member 44 is spandex, and is coated with a hot-melt adhesive using the V-slot method. A stretchable nonwoven cloth or the like may be used for the central elastic member 44. The elastic member-covering sheet 43 is configured by a nonwoven cloth or other sheet, and, in the present embodiment, an SMS nonwoven cloth (hydrophobic), having a basis weight of 15 $g/m^2$, made of polypropylene is used.

Examples of the material of the central elastic member 44 include synthetic rubber such as styrene-butadiene, butadiene, isoprene, and neoprene, natural rubber, EVA, elastic polyolefin, spandex, and foamed polyurethane. In addition, as the material of the central elastic member 44, an elastic sheet such as a stretchable nonwoven cloth formed by mixing and then stretch-processing urethane, polystyrene or other elastomer fiber with stretchable polyolefin, polyester, or other thermoplastic fiber, may be used.

A side-end elastic member 49 is joined by an adhesive between the absorber backside-covering sheet 30 and the sidesheet 60 at both sides in the widthwise direction of the absorber. The side-end elastic member 49 is arranged along the front-back direction, and is arranged so as to straddle the urination region S3 and a portion of the rear waistline region S2.

Means of fixing the side-end elastic member 49 includes a hot-melt adhesive or the like. The side-end elastic member 49 according to the present embodiment uses spandex and is directly coated with a hot-melt adhesive using the V-slot method. The side-end elastic member 49 according to the present embodiment is extended and fixed in threes on both the right and left sides at a thickness of 780 dtex and an extension rate of 2.3 times.

The side-end elastic member 49 and the front leg hole elastic member 5F, as well as the side-end elastic member 49 and the rear leg hole elastic member 5R, are arranged substantially in respective series when seen in a plan view. By this arrangement, the materials can stretch around leg holes, thereby having an effect of making leg holes more fitting and preventing a diaper from slipping off or leaking.

As for each member constituting the aforementioned disposable diaper 1, the material described in Japanese Unexamined Patent Application Publication No. 2006-346439, for example, may be used.

(2) Configuration of the absorbent sheet

Next, a description of the absorbent sheet 90 arranged on the skin contact surface side of the absorber 40 in the rear waistline region S2 shall be provided. The absorbent sheet 90 is a composite sheet in which the absorbent polymer is sandwiched between two liquid-permeable sheets 92 and 93. For the liquid-permeable sheets 92 and 93, for example, a spun bond nonwoven cloth, a point-bond nonwoven cloth, an air-through nonwoven cloth, an SMS nonwoven cloth, or the like, may be used. The liquid-permeable sheets 92 and 93 need to be permeable to urine, and therefore a hydrophilization treatment is preferably performed. However, when the absorbent sheet 90 is arranged in the non-skin surface side from the absorber 40, the sheet at the non-skin surface side out of the liquid-permeable sheets 92 and 93, may be liquid impermeable. In the present example, a 25 $g/m^2$ air-through nonwoven cloth to which a hydrophilization treatment has been performed is used as the liquid-permeable sheets 92 and 93.

The absorbent polymer may be sandwiched between three or more liquid-permeable sheets to make the absorbent sheet, or a single liquid-permeable sheet may be folded over and sandwich the absorbent polymer to make the absorbent sheet.

Examples of the absorbent polymer include starch-based, acrylic acid-based, or amino-acid based particulate or fibrous polymers. The particle diameter of the absorbent polymer is preferably not finer than the intervals between fibers in the liquid-permeable sheets 92 and 93, in order that the absorbent polymer is prevented from spilling after the absorbent polymer has been sandwiched between the two liquid-permeable sheets 92 and 93.

The absorbent polymer is a polymer which absorbs water and is, for example, a highly polymerized absorbent polymer having a three-dimensional network structure in which a water-soluble polymer has been moderately cross-linked. Such a highly polymerized absorbent polymer absorbs water of several hundred to a thousand times the volume before the absorbent polymer absorbs water; however, the absorbent polymer is substantially water-insoluble, and water, once absorbed, will not leave, even when a certain amount of pressure is applied.

After the absorbent polymer has been sandwiched with the two liquid-permeable sheets 92 and 93, the two liquid-permeable sheets 92 and 93 are joined, thus making the absorbent sheet 90. The regions where the absorbent polymer has been arranged between the two liquid-permeable sheets 92 and 93 are absorbent polymer arranged regions 96, and the regions where the absorbent polymer has not been arranged between the two liquid-permeable sheets 92 and 93 and the two liquid-permeable sheets 92 and 93 are joined are absorbent polymer non-arranged regions 97. Fig. 2 shows the absorbent polymer arranged regions 96 with diagonal lines.

It is noted that, an inner end in the front-back direction of the absorbent polymer arranged region in the present embodiment is an inner end in the front-back direction of an absorbent polymer arranged region 96 that is arranged in the innermost side in the front-back direction out of a plurality of absorbent polymer arranged regions 96. Fig. 2 shows an inner end in the front-back direction 96A, of the absorbent polymer arranged regions 96.

Arranging absorbent polymer non-arranged regions 97 between the absorbent polymer arranged regions 96 allows urine to flow through the absorbent polymer non-arranged regions 97 and to be absorbed by the absorber 40 even in a case where the absorbent polymer of the absorbent polymer arranged regions 96 has absorbed urine and becomes fully swollen, thus becoming unable to further absorb urine. It is noted that for an absorbent article which does not have to be in use until the absorbent polymer swells to the full, it is unnecessary to provide an absorbent polymer non-arranged region for the absorbent sheet.

The shape of the absorbent polymer arranged region 96 in the planar direction is convex in the direction from the front waistline region S1 to the rear waistline region S2 in the front-back direction of the absorbent article 1, and is substantially V-shaped extending in the widthwise direction of the absorbent article 1. Specifically, the shape of the absorbent polymer arranged region 96 in the planar direction is substantially V-shaped where the vertex faces the direction from the front waistline region S1 toward the rear waistline region S2 in the front-back direction of the absorbent article 1.

The substantially V-shape of the absorbent polymer arranged region 96 is easily bent at the center in the widthwise direction. Therefore, when the absorbent sheet is arranged along the ventral of the wearer, the absorbent sheet is easily arranged so as to include male genitalia. Further, the absorbent polymer arranged region 96 takes a substantially V-shape along the front groin portion of the wearer, and thus, even after the absorbent polymer absorbs urine, the absorbent polymer arranged region 96 fits the groin portion of the wearer, making it less likely that a gap is generated between the wearer and the absorbent article 1. In addition, even when the legs are moved, the movement of the legs is not inhibited.

Further, giving the absorbent polymer arranged regions 96 a substantial V-shape or substantially arc shape and lining them up in a row in the front-back direction makes it possible to keep the total widthwise length of the absorbent polymer non-arranged regions 97 substantially constant.

The surface of the liquid-permeable sheets 92 and 93 where the absorbent polymer is sandwiched is provided with a plurality of adhesive units (not shown) extending in the front-back direction and lined up in the widthwise direction. The adhesive unit can be formed of a heat seal, a sonic seal, an adhesive, or the like, and specifically, for example, the adhesive unit can be formed by coating a hot-melt adhesive in streaks.

Adhesive units according to the present embodiment extend in the front-back direction, and are arranged in plurality in the widthwise direction, that is, the adhesive units are arranged in streaks. Since the permeability of urine in the adhesive units declines easily as compared to the other regions, it is desirable to provide the adhesive units in streaks so as to provide a region where the adhesive units do not exist. It is noted that as long as a coating method is a method in which a region with no adhesive units can be provided, specifically, as long as a coating method is a method in which the adhesive units can be arranged in discontinuation, the method is not limited to a streak-pattern coating method, and a non-contact coating method such as omega coating may be used.

It is noted that since the liquid-permeable sheets 92 and 93 are in a wet state when the absorbent polymer is swollen, the adhesive is preferably of a type which exhibits strength even in a wet state.

Furthermore, in a case where a heat seal is used to join the liquid-permeable sheets 92 and 93, a greater constancy of the line pressure, in the widthwise direction, of the heat seal corresponds to an overall more even sealing strength. Therefore, it is preferable that the line pressure in the widthwise direction of the heat seal be constant. In order to even out the line pressure in the widthwise direction of the heat seal, it is desirable to keep the total widthwise length of the sealed region constant.

The adhesive units are provided intermittently in the absorbent polymer arranged region 96, and thereby it is possible to prevent the absorbent polymer from being shifted within the absorbent polymer arranged region 96.

The adhesive units may also be provided to the surface of one liquid-permeable sheet 92 or 93, out of the liquid-permeable sheets 92 and 93, where the absorbent polymer is sandwiched. Further, the distance between mutually adjacent adhesive units of the adhesive units provided in the liquid-permeable sheet 93 at the side of the absorber 40 may be configured to be shorter than the distance between the adhesive units provided in the liquid-permeable sheet 92 at the side of the topsheet 10. With such a configuration, the absorbent polymer can be substantially evenly fixed to the liquid-permeable sheet 93 at the side of the absorber 40.

The joining of the two liquid-permeable sheets 92 and 93 requires a strength that can withstand the expansion force caused by the swelling of the absorbent polymer. In some cases, when, for example, the swelling of the absorbent polymer causes the two liquid-permeable sheets 92 and 93 to be peeled away from each other, a gap is created between the two liquid-permeable sheets 92 and 93, into which the absorbent polymer enters, thus eliminating the absorbent polymer non-arranged region 97.

Furthermore, by providing a color to the adhesive constituting the adhesive units, streak-pattern adhesive units colored to be blue or another color can be provided in the liquid-permeable sheets 92 and 93, and the outer appearance can be improved by providing a striped pattern.

Desirably, because the absorbent polymer swells upon absorbing urine, the absorbent polymer in the absorbent polymer arranged regions 96 secures sufficient volume between the two liquid-permeable sheets 92 and 93 so as to allow the absorbent polymer to swell sufficiently. When the absorbent polymer is too densely packed in between the two liquid-permeable sheets 92 and 93, the absorbent polymer is unable to swell completely, and in some cases it is not possible to fully make use of the absorption capacity of the absorbent polymer.

When the number of the absorbent polymer arranged regions 96 in the absorbent sheet 90 is increased, the area of the absorbent polymer non-arranged regions 97 increases; however, the total volume of the absorbent polymer in the absorbent sheet 90 decreases, and so the amount of urine that can be absorbed by the absorbent sheet 90 reduces. On the other hand, when the number of the absorbent polymer arranged regions 96 in the absorbent sheet 90 is reduced, the total volume of the absorbent polymer in the absorbent sheet 90 is increased and the amount of urine which can be absorbed by the absorbent sheet 90 is increased, however, the area of the absorbent polymer non-arranged regions 97 is decreased, and in some cases, after the absorbent polymer has swollen, urine will no longer permeate through the absorbent sheet 90 and be absorbed into the absorber 40.

It is necessary to select the number of absorbent polymer arranged regions 96 in consideration of a balance between the amount of urine and the permeability of urine at the absorbent sheet 90 after the absorbent polymer is swollen, and also of a capacity after the swelling based on the kinds and input amounts of the absorbent polymer. For example, when the absorbent sheet is 160 mm × 130 mm in size, 1.6 g of absorbent polymer which absorbs 60 g of physiological salt solution per 1 g, is arranged separately at three absorbent polymer arranged regions, thus providing an absorbent sheet having a preferable balance between urine absorption properties and urine permeability after the absorbent polymer is swollen.

Particularly, when the disposable diaper is used by children or men who show a large tilting of the body in the lying posture or who are more prone to lying face down, it is desired to provide the absorbent polymer arranged regions 96 in the region across the front waistline region S1 from the center in the front-back direction of the urination region S3, and it is preferable to arrange the absorbent polymer arranged regions 96 in at least a part of the front waistline region S1. The absorbent polymer arranged regions 96 are provided in the front waistline region S1, thus making it possible to secure the absorption capacity in the front waistline region S1 and prevent leakage in the front waistline region S1.

The absorbent sheet 90 may be arranged on the non-skin surface side of the absorber 40, however, is preferably arranged on the skin surface side. In a state where the body fluid is absorbed by an absorber, when the body pressure is applied to the absorber, the urine absorbed into the absorber 40 oozes out due to the body pressure, and may flow back to the side of the wearer. However, by providing the absorbent sheet, the body fluid oozing out from the absorber can be hindered from reaching the side of the skin of the wearer. Thus, even after the wearer has urinated, the surface that is in contact with the skin does not dampen, and rashes on the skin of the wearer caused by the excreted urine can be restrained.

The uplifting effect of the leg hole elastic member causes such a configuration that the absorber will not be separated from the skin even in a case where the absorbent sheet 90 absorbs fluid and becomes heavy. Accordingly, it becomes less likely that a gap is created between the absorber and the skin, in particular, to reduce the occurrence of leakage caused by running along the skin. In a case where the absorber is configured so as not to be separated from the skin, irritation of the skin is particularly prone to occur when body fluid oozing out from the absorber 40 is likely to reach the skin of the wearer. Therefore, it is more preferable to arrange the absorbent sheet 90 on the skin surface side of the absorber 40.

Furthermore, in the present embodiment, the uplifting effect of pulling up the absorbent sheet toward the side of the wearer by the leg hole elastic members is exhibited; however, the configuration is not limited thereto, and the uplifting effect can be realized by another configuration as well. Furthermore, the elastic member that pulls up the absorbent sheet is desired to be provided close to the absorbent sheet. By providing the elastic member close to the absorbent sheet, the absorbent sheet can be effectively pulled up toward the side of the wearer.

(3) Structure of the absorber

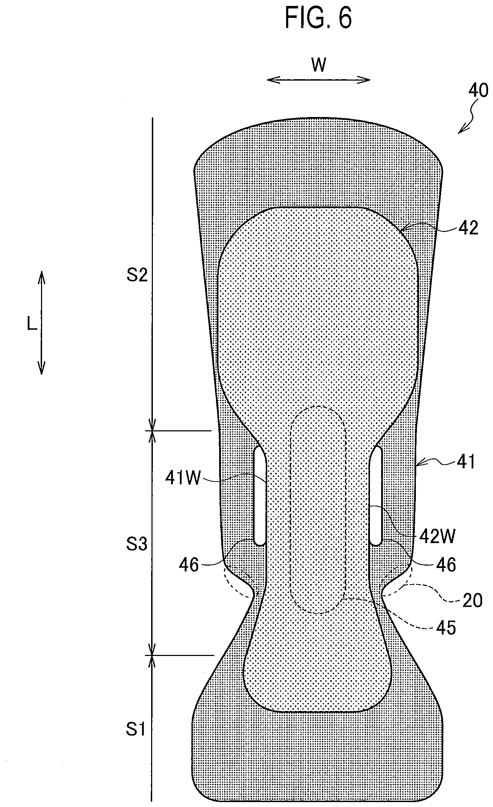

Fig. 6 is a plan view of the absorber 40. As shown in Fig. 6, the absorber 40 has a first layer 41, and a second layer 42 on top of the first layer 41. The first layer 41 is positioned at the non-skin contact surface side of the wearer, and the second layer 42 is positioned at the skin contact surface side of the wearer. The first layer 41 protrudes outward in the front-back direction and the widthwise direction from the second layer 42.

The first layer 41 and the second layer 42 are configured by cotton-like pulp and superabsorbent polymer (SAP). The absorber 40 can be formed by mixing, for example, 0 to 500 g/m$^2$ of pulp and 0 to 500 g/m$^2$ of SAP.

The first layer 41 according to the present embodiment is formed by mixing 280 g/m2 of pulp and 170 g/m$^2$ of SAP. The second layer 42 according to the present embodiment is formed by mixing 260 g/m$^2$ of pulp and 160 g/m$^2$ of SAP.

The first layer 41 is formed with the central aperture 45 and a pair of the side slits 46. The central aperture 45 is formed in the central part in the widthwise direction W. The length of the central aperture 45 in the front-back direction is longer than the length of the side slits 46 in the front-back direction. In the present embodiment, the central aperture is 40 mm wide and each of the side slits 46 is 10 mm wide.

The front end of the central aperture 45 is adjacent to the absorbent sheet. The central aperture 45 and the side slits 46 have a vertically long shape extending along the front-back direction. The central aperture 45 and the side slits 46 are formed extending in the front-back direction in the urination region S3, and function as guide portions that guide body fluid absorbed by the absorber 40 of the urination region S3 to the absorbent sheet 90 of the front waistline region S1. By providing the guide portions, the urine excreted in the urination region is directed to an absorbent sheet having a large amount of SAP, and the urine can be concentratedly held by the SAP of the absorbent sheet.

The front end of the central aperture 45 may be arranged so as to overlap the absorbent sheet 90, or may be arranged so as not to overlap but rather close to the absorbent sheet. Also, the absorbent sheet may be arranged to be on an extension line that extends towards the front of the central aperture.

By arranging the central aperture and the absorbent sheet as described above, the body fluid that is pulled in by the absorber is more easily guided to the front waistline region S1 smoothly through the central aperture 45 and the side slits 46. Directing the body fluid to the absorbent sheet 90 arranged in the front waistline region S1 makes it possible to preferentially concentrate the body fluid in a portion having high absorption capacity.

Consequently, absorption capacity can be effectively used and the absorption capacity of the entire absorbent article can be improved. When the excreted urine is concentratedly held in a crotch unit, the absorber swells in the crotch unit, it becomes more difficult to close the legs, greater discomfort occurs, and it becomes more difficult to walk. However, by directing the urine to, and concentratedly holding the urine in, the distally positioned absorbent sheet separated from the urination region, it is possible to improve absorption efficiency and reduce leakage even while reducing the discomfort caused by the swelling of the absorbent polymer after urine absorption in the crotch unit and caused by an increase in the thickness of the absorbers.

In the present example, a distance of about 10 mm in a plan view exists between the inner end in the front-back direction 96A of the absorbent polymer arranged regions 96 of the absorbent sheet 90 and the central aperture 45. The region between the inner end in the front-back direction 96A of the absorbent polymer arranged regions 96 and the end of the center slit is termed an intermediate region 110. The intermediate region 110 is configured to guide the body fluid to the absorbent sheet 90 in which the absorbent polymer is arranged. The intermediate region is desirably configured to be capable of repeatedly diffusing the body fluid, and specifically, the weight ratio of the SAP is desirably less than 80%. The weight ratio of the SAP in the intermediate region according to the present embodiment is 38%.

The guide portions according to the present embodiment include openings such as slits formed at the absorber, but are not limited to this configuration. The guide portions may be configured by, for example, a low basis weight region having a lower basis weight than that of the surrounding at the absorber, or a compressed portion compressed in the thickness direction of the absorber.

For example, when the guide portions are configured by low basis weight regions, it is better in a sense that grooves (passages for fluid) are not blocked even at the time of repeated absorption since the basis weight of the absorbent polymer in the low basis weight region is lower than the basis weight of the absorbent polymer at the compressed portion.

Moreover, when the guide portions are configured by the compressed portions, the guide portions are superior in that the units have a high density and thus have excellent diffusibility, so that the units can diffuse fluid well, in particular, even in the direction against gravity compared with a case where the guide portions are configured by a low basis weight region. The low basis weight region in the present invention is a region having a basis weight ratio of at most 50% relative to the average basis weight of the absorbent layer constituting the absorber of the surrounding.

The basis weight can be measured by, for example, the following method. Firstly, markings are made on the topsheet at the size of 10-mm width and 50-mm length in the region to be measured of the product, and the absorber is taken out by sheet member at the size to measure the initial weight. Set this weight as A (g).

Next, wash the sheet members (such as the topsheet, a second sheet, and a backside-covering sheet) that are cut together with an organic solvent such as toluene, measure the weight after drying, and then determine the total weight of the sheet members. Set this weight as B (g).

Using the initial weight (A) and the sheet member weight (B), the basis weight of the absorber is calculated by "(A-B)/Sampling size". The basis weight X in the present embodiment can specifically be calculated by $(A - B)/(10 \times 50/1000/1000)$ (g/m$^2$). The aforementioned weight is a weight including the hot-melt adhesive; however, the weight of the hot-melt adhesive is extremely small as compared to the weight of the absorber and the like, and thus, may be ignored.

The basis weight ratio is calculated on the basis of the basis weight of the absorber as determined by the above method.

$$\text{Basis weight ratio} = (\text{basis weight of the absorber in the low basis weight region})/(\text{basis weight of the absorber in the surrounding}) \times 100.$$

The low basis weight region is a region where the basis weight ratio is at most 50 (%).

Specifically, the low basis weight region is understood to be where the following condition applies.

$$\text{(Basis weight of the absorber in the low basis weight region)/ (basis weight}$$
$$\text{of the absorber in the surrounding)} \times 100 \leq 50 \; (\%)$$

In a case where the low basis weight region is at a point where there is no overlap with the other layers constituting the absorber (for example, a region which is formed in the first layer and is not covered with the second layer), the aforementioned method is used to determine the basis weight of the layer of the low basis weight region to be measured and to determine the basis weight of the surrounding of the layer.

Furthermore, in a case where the low basis weight region is at a point where there is overlap with the other layers (for example, a region which is formed in the first layer and is covered with the second layer), it is difficult to measure the basis weight of the low basis weight region of a single layer, and therefore a basis weight X1 of the portion of the low basis weight region to be measured where the layers overlap is determined, a basis weight X2 of the absorber in the overlapping portion of the surrounding is then determined, and a basis weight X3 of the portion of the single layer, where there is no overlap, is determined, whereby the basis weight of the low basis weight region is measured.

Additionally, in such a case, (1) when the layer having the low basis weight region and the above-described single layer are matching, it can be assumed that the low basis weight region satisfies the condition (X1 - X2 + X3)/X3 x 100 ≤ 50 (%).

On the other hand, (2) in a case where the layer having the low basis weight region and the above-described single layer do not match, it can be assumed that the low basis weight region satisfies the condition (X1 - X3)/(X2 - X3) × 100 ≤ 50 (%).

That is, depending on the structure of the low basis weight region, the basis weight can be measured by the above-described three methods.

Additionally, as the central aperture 45 is formed, a central part of the absorber 40 can easily bend convexly in the inner direction IN towards a wearer. The side slit 46 is formed at the outer side in the widthwise direction with respect to the central aperture 45. The side slit 46 has a vertically long shape extending along the front-back direction. The pair of side slits 46 are formed on the absorber 40 along the front-back direction convexly in the outer direction OUT, that is, such that the absorber 40 bends convexly inverse to the central aperture 45.

In the urination region S3, an outer end 42W of the second layer 42 in the widthwise direction has overlap with an inner end 41W of the side slit 46 of the first layer, and is arranged along the front-back direction. The outer side in the widthwise direction of the absorber 40 beyond the outer end 42W is configured by the first layer 41 only, and the inner side beyond the outer end 42W is configured by the first layer 41 and the second layer 42, excluding the portion where the central aperture 45 has been formed. Accordingly, the absorber 40 changes in rigidity and thickness at a boundary formed of the inner end 41W of the first layer 41 and the outer end 42W of the second layer 42. In the present embodiment, the absorber bends at a boundary formed of the outer end 42W of the second layer where the rigidity and other properties change.

Causing the topsheet 10 and the absorber backside-covering sheet 30 to be pasted together and fixed at the side slit 46 makes it possible for the absorber to bend without losing shape.

In the first layer, a constriction portion 20 that is constricted to the inner side in the widthwise direction in the urination region S3, is formed. The constriction portion 20 is a portion having the width formed to be the shortest in the first layer. The constriction portion functions as a deformation restraining unit that restrains deformation in the urination region from being propagated to the absorbent sheet. Fig. 6 shows constriction portions 20 surrounded by dotted lines. Further, also at the outer ends in the widthwise direction of the second layer, the width is formed to be gradually shorter along the constriction portion 20.

The second layer 42 is substantially in an hourglass shape. The constriction portion 20 is formed by including a portion having the shortest width in the first layer. The portion having the shortest width in the first layer is arranged to the rear of the inner end in the front-back direction 96A of the absorbent polymer arranged regions 96. Further, the portion having the shortest width in the second layer is arranged in the inner side in the widthwise direction of the constriction portions 20 and to the rear of the inner end in the front-back direction 96A of the absorbent polymer arranged regions 96. Since the constriction portion 20 as the deformation restraining unit and the portion having the shortest width in the second layer are provided to the rear of the inner end in the front-back direction 96A of the absorbent polymer arranged regions 96, it is possible to restrain the deformation of the absorber in the urination region from being spread to the inner end in the front-back direction 96A of the absorbent polymer arranged regions 96.

Since the deformation of the urination region does not directly spread to the inner end in the front-back direction 96A of the absorbent polymer arranged regions 96, it is possible to make it less likely that wrinkles are generated in the absorbent polymer arranged regions 96. Since swelling of the absorbent polymer is not inhibited, the absorption efficiency can be improved and an area capable of absorbing is increased, thus making it possible to reduce leakage over a wide range and to provide more comfortable feeling at the time of wearing by the improvement of absorption performance.

(3) Changes in shape of absorber

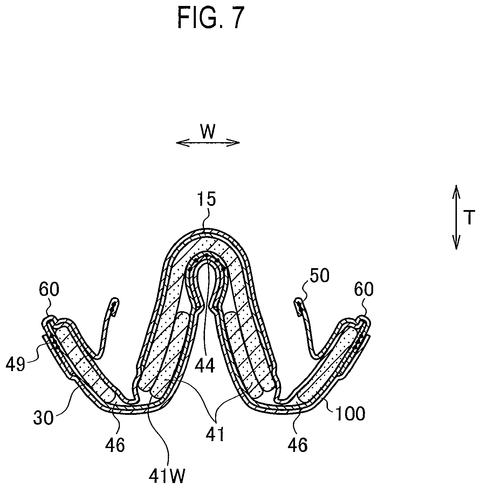

Fig. 7 is a cross-sectional view (with reference to an X1-X'1 line of Fig. 1) that schematically illustrates the state when the disposable diaper 1 is worn. When the disposable diaper 1 is worn, the urination region S3 of the absorber comes up against the crotch of the wearer. By the legs and the like of the wearer, force is applied on the absorber from the outer side in the widthwise direction toward the inner side in the widthwise direction. The absorber 40 bends at the side slits 46 as well as the central elastic member 44 and the central aperture 45, and the cross-sectional shape of the disposable diaper 1 along the widthwise direction W is deformed in a wavelike manner. Thus, the urination region S3 of the absorber 40 turns into a regularly folded-up condition.

The top surface of the absorber 40, which becomes convex in the inner direction IN due to the central elastic member 44, comes into contact with the crotch portion of the wearer. The portion where the convex portion, caused by the curving unit, is formed is configured by only the second layer 42, and is relatively thinner. On the other hand, the first layer 41 and the second layer 42 overlap between the convex portion caused by the curving unit and the convex portion caused by the side slit, this overlap being relatively thicker and more rigid. The convex portion formed of the curving unit can be supported by the portion having a high rigidity between the curving unit and the side slit, and the stability of the convex shape formed of the curving unit can be improved.

When the wearer closes both legs, with respect to the cross-sectional shape of the disposable diaper 1, the absorber is folded over at the curving unit and the side slits and is compactly arranged below the crotch portion in a state of close mutual contact.

At such a time, the curving unit formed of the central elastic member 44 and the central aperture 45 is positioned so as to be in contact with the crotch portion of the wearer. On the other hand, the curving units formed of the side slits 46, project towards the non-skin contact surface side and are at a position where it is difficult to contact with a wearer's excretion portion.

Because the absorber is in close contact at the crotch portion of the wearer, leakage of the body fluid can be prevented even in a case where urine is slowly excreted, which would run along the skin. In the folded state, a concavity extending in the front-back direction is formed at the curving unit of the portion of the absorber separated from the skin, and therefore the body fluid can be diffused outward in the front-back direction and side leakage can be prevented.

The absorber is folded up at the central aperture 45 and the side slits 46 formed in the absorber so that the absorber 40 can bend more easily even when swollen by absorbing liquid compared with a case where the absorber 40 has a portion protruded by forming a thinner portion. The cross-sectional shape obtained when the disposable diaper 1 is worn and the absorber 40 is deformed is a tapered shape which narrows from the non-skin contact surface side to the skin contact surface side. Accordingly, the disposable diaper 1 can be easily fitted into the gap in the crotch portion of the wearer, and is less prone to cause discomfort.

The convex portion formed of the curving unit is configured only by the second layer 42, and is thinner than the portion configured by laminating the first layer 41 and the second layer 42. In other words, due to a thin and high structure, the convex portion formed of the curving unit can be inserted easily in the thin gap of the crotch portion, and easily adheres on to the excretion portion. Accordingly, because there is close contact between the urine outlet and the absorber, excreted urine can be absorbed rapidly. Because the folding is such that the absorber is thin in the portion close to the skin of the crotch portion of the wearer and is thicker at the portion farther from the skin, fitting can be done without discomfort.

The structure with which it is easier to enter into the thin gap in the crotch portion of the body makes it possible to reduce the distance between the central aperture 45 and the urination portion. Reducing the distance between the central aperture 45 and the urination portion makes it possible to diffuse excreted urine in the front-back direction by the central aperture 45, and guide the urine to the absorbent sheet 90 of the front waistline region S1. Unencumbered guidance of the body fluid to the absorbent sheet 90 makes it possible to preferentially concentrate the body fluid at the portion having high absorption capacity and makes it possible to make use of the absorption capacity more efficiently, whereby absorption performance can be further enhanced.

With respect to a method of producing the absorbent article thus configured, the absorbent article can be produced by a method comprising, for example, a step of forming a first layer of the absorber, a step of forming a second layer of the absorber, a step of conjoining the first layer and the second layer, and a step of using a conveyor belt or the like to convey the absorber and the like and, during the process of conveyance, joining the absorbent sheet, the topsheet, and other sheet materials. Production involving other steps is also possible in accordance with a known production method.

When an aperture and side slits are provided at both the first layer and the second layer, there may be positional deviation in case of laminating the first layer and the second layer. When the positional deviation occurs in, for example, the widthwise direction, the width of the pair of side slits arranged on the right and left is narrowed, regular deformation becomes impossible, and the absorber has a right-left imbalance, which may exert an adverse effect on absorption and on the feeling of comfort at the time of wearing. However, by providing an aperture or side slits to at least one of the first layer and the second layer, the positional deviation of the side slits or the like can be prevented.

<Second embodiment> (not according to the invention)

A description of the configuration of an absorber of a disposable diaper according to modifications shall next be provided, with reference to the accompanying drawings. In the following embodiment, the same components as those in the above-described first embodiment will be assigned with the same reference numerals, and redundant description thereof will not be repeated.

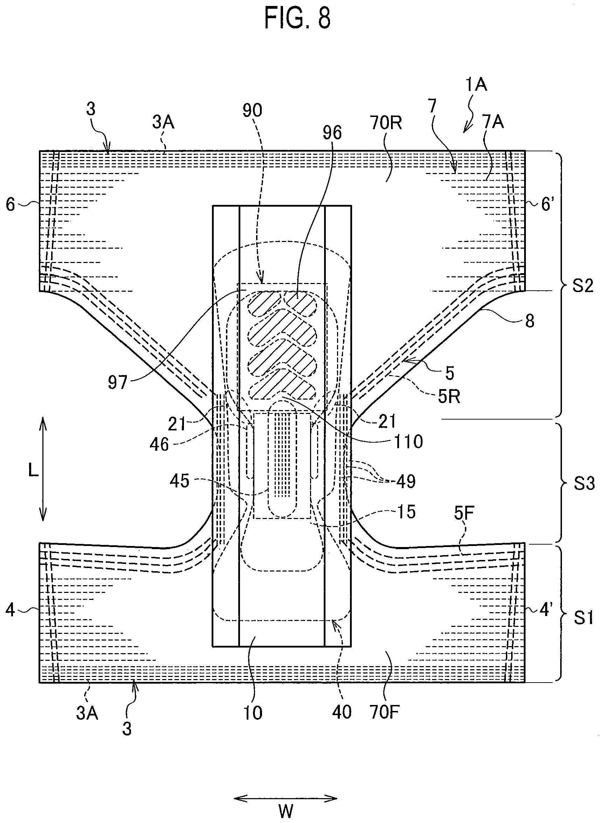

Fig. 8 is a developed plan view of a disposable diaper 1A according to a second embodiment. In the disposable diaper 1A according to the second embodiment, the absorbent sheet 90 is provided in the rear waistline region S2. Although the shape of the absorber is the same as that of the first embodiment, the configuration of the deformation restraining unit is different from that of the first embodiment. The outer end in the widthwise direction of the second layer in the rear waistline region functions as a deformation restraining unit 21.

The deformation restraining unit 21 is to the rear of the side slits and is arranged from the urination region S3 to the rear waistline region S2. Moreover, the deformation restraining unit 21, from the rear end of the narrowest portion of the second layer 42 towards the rear, is arranged to extend from the inner side in the widthwise direction toward the outer side in the widthwise direction. In a region to the front of the deformation restraining unit 21, which is a region in which the side slits are arranged, only the first layer 41 is arranged, while in a region to the rear of the deformation restraining unit 21, the first layer 41 and the second layer overlap each other, causing a difference in rigidity of the absorber at the deformation restraining unit. Thus, it becomes less likely that deformation to the front of the deformation restraining unit 21 is spread to the rear in which the absorbent sheet is arranged.

By arranging the absorbent sheet 90 in the rear waistline region S2, it is possible to effectively absorb the body fluid that is guided from the urination region to the rear waistline region especially in the lying posture on one's back and on one's side. It is noted that, preferably, the absorbent sheet 90 is arranged in at least a part of the rear waistline region S2, over the rear waistline region S2 from the center in the front-back direction of the urination region S3. Leakage in the rear waistline region can be prevented by arranging the absorbent polymer concentratedly in the rear waistline region to improve absorption capacity of the rear waistline region S2.

The shape of the absorbent polymer arranged region 96 in the planar direction is a convex shape having a vertex facing the direction from the front waistline region S1 to the rear waistline region S2, and is substantially V-shaped extending in the widthwise direction. Specifically, the shape of the absorbent polymer arranged region 96 in the planar direction is substantially V-shaped where the vertex faces the direction from the front waistline region S1 to the rear waistline region S2.

The body pressure in a sitting position exerted on the absorbent sheet is exerted forward from the center in the widthwise direction toward the outer side in the widthwise direction. The substantially V-shape of the absorbent polymer arranged region 96 is a shape along the portion on which the body pressure is exerted. The absorbent polymer is arranged in the region on which the body pressure is exerted in the sitting position, thereby making it possible to hold the body fluid on the portion on which the body pressure is exerted and to prevent backflow when the body pressure is exerted.

Provided that the absorbent polymer non-arranged regions exists, the shape of the absorbent polymer arranged region is not limited to be substantially V-shaped. For example, the absorbent polymer arranged region may be in the shape of an arc, a circle, a rectangle, a triangle, and the like.

Further, the substantial V-shape of the absorbent polymer arranged region 96 is a shape that runs along the buttocks of the wearer, and therefore the absorbent polymer arranged region 96 fits the buttocks of the wearer, making it less likely that a gap is generated between the wearer and the absorbent polymer arranged region 96.

<Third embodiment> (not according to the invention)

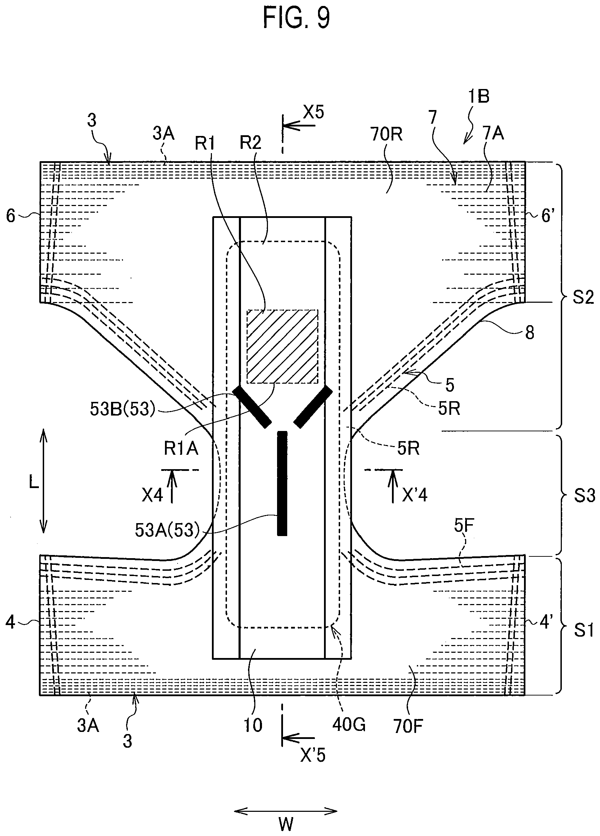

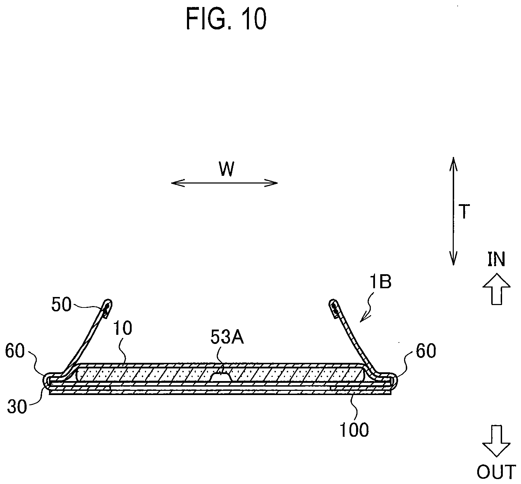

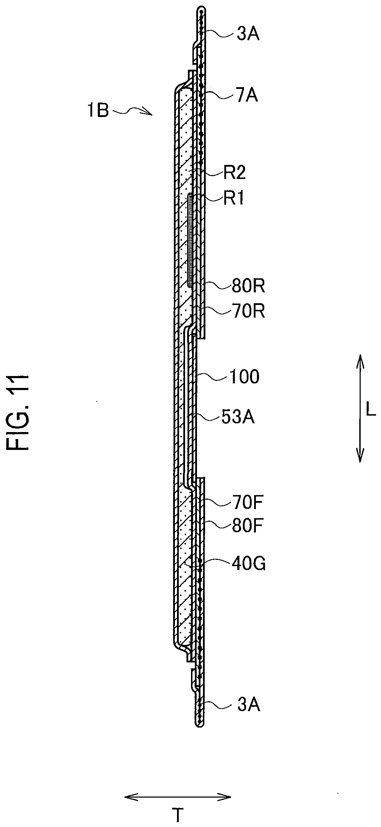

Fig. 9 is a developed plan view of a disposable diaper 1B according to a third embodiment. Fig. 10 is a widthwise cross-sectional view of the disposable diaper 1 along an X4-X'4 line shown in Fig. 9. Fig. 11 is a cross-sectional view in a front-back direction of the disposable diaper 1 along an X5-X'5 line shown in Fig. 9. An absorber 40G of the disposable diaper 1B according to the third embodiment is different from the absorber according to the first embodiment and the second embodiment, configured by one layer, and has a first region R1 having an absorbent polymer and a second region R2 having a lower basis weight of an absorbent polymer than that of the first region R1.

The first region R1 has a relatively higher basis weight of an absorbent polymer of the entire absorber and is provided on the side of an exterior sheet in the front waistline region. The region surrounding the first region R1 at the absorber is the second region. The second region R2 may be configured to have a lower basis weight of the absorbent polymer than the first region R1, or may be a region which does not have the absorbent polymer (the basis weight of the absorbent polymer is 0 g/m$^2$).

Fig. 9 shows an inner end in the front-back direction R1A in the first region R1 in the present embodiment. It is noted that, in an embodiment in which a plurality of first regions are provided, an inner end in the front-back direction of the first region R1 arranged in the innermost side in the front-back direction out of the plurality of the first regions R1, is shown.

Further, in the absorber 40G, a compressed portion 53 in which the absorber is compressed in a thickness direction is formed. The compressed portion 53 is formed with the absorber 40G compressed toward the inner direction from the outer direction, and concaved toward the inner direction.

The compressed portion 53 has a first compressed portion 53A arranged in the center in the widthwise direction of the urination region and a second compressed portion 53B arranged to the rear of the first compressed portion. The first compressed portion 53A is formed along the front-back direction and functions as a curving unit that is formed such that the absorber is bent convexly toward the wearer, and functions as a guide portion that guides the body fluid from the urination region S3 to the first region R1. The second compressed portion 53B extends, toward the rear, from the inner side in the widthwise direction to the outer side in the widthwise direction and functions as a deformation restraining unit that restrains deformation of the urination region S3 from being spread to the rear waistline region S2.

Since the absorber is deformed in a convex shape toward the inner direction by the first compressed portion, the absorber is arranged in close contact with the wearer in the urination region. On the other hand, the absorber to the rear of the second compressed portion is planarly arranged since deformation of the urination region is less likely to be propagated by the second compressed portion as the deformation restraining unit. The first region R1 is arranged to the rear of the second compressed portion and arranged in a state of facing the wearer.

<Fourth embodiment> (not according to the invention)

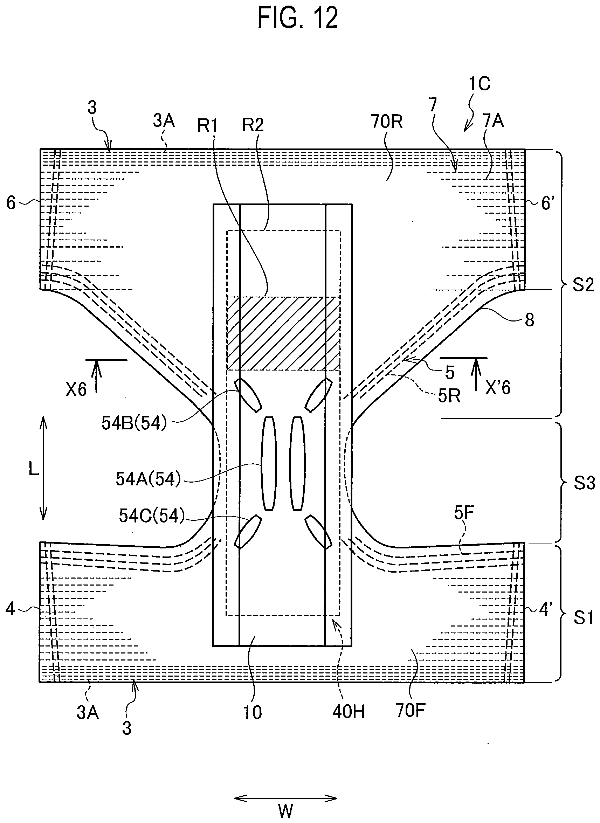

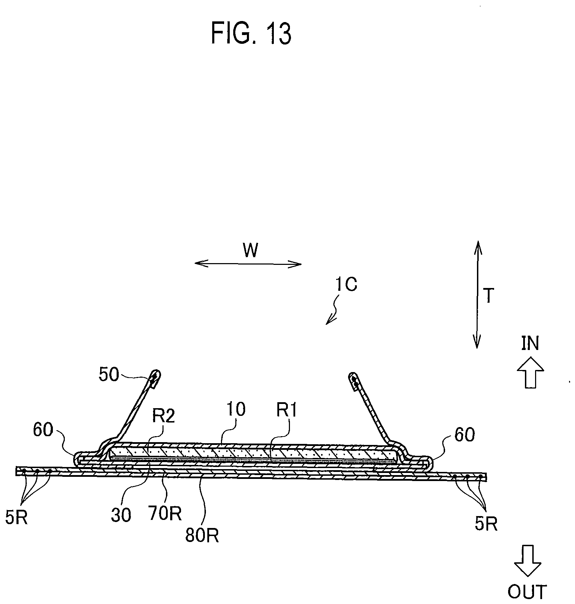

Fig. 12 is a developed plan view of a disposable diaper 1C according to a fourth embodiment. Fig. 13 is a widthwise cross-sectional view of the disposable diaper 1 along an X6-X'6 line shown in Fig. 12. An absorber 40H of the disposable diaper 1C according to the fourth embodiment is configured by one layer, has a first region R1 having an absorbent polymer and a second region R2 having a lower basis weight of an absorbent polymer than that of the first region, and slits are formed on the absorber 40H.

A slit 54 has a pair of first slits 54A arranged along the front-back direction in the urination region, a pair of second slits 54B arranged to the rear of the first slits 54A, and a pair of third slits 54C arranged to the front of the first slits 54A.

The first slits 54A are formed along the front-back direction and function as guide portions that guide the body fluid from the urination region to the first region. The second slits 54B extend, toward the rear, from the inner side in the widthwise direction to the outer side in the widthwise direction and function as deformation restraining units that restrain deformation of the urination region from being spread to the rear waistline region S2. The third slits extend, toward a front, from the inner side in a widthwise direction to the outer side in a widthwise direction.

The absorber to the rear of the second slits is planarly arranged since deformation of the urination region is less likely to be propagated by the second slits as deformation restraining units. The first region is arranged to the rear of the second slits of the absorber and arranged in a state of facing the wearer. The body fluid excreted to the urination region is guided to the first region through the first slits and absorbed in the first region.

<Fifth embodiment> (not according to the invention)

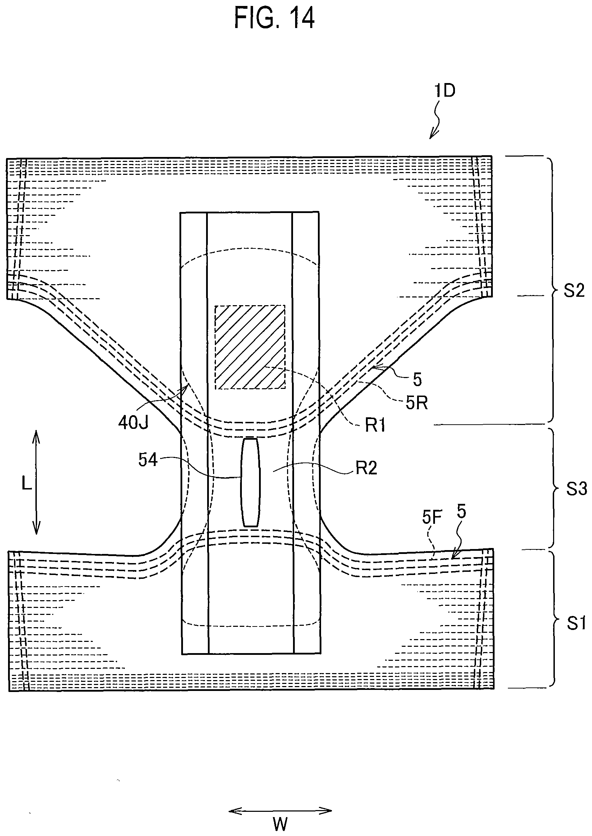

Fig. 14 is a developed plan view of a disposable diaper 1D according to a fifth embodiment. An absorber 40J of the disposable diaper 1D according to the fifth embodiment is configured by one layer, has a first region R1 having an absorbent polymer and a second region R2 having a lower basis weight of an absorbent polymer than that of the first region, and the slit 54 is formed on the absorber 40J.

The first region R1 is arranged at the skin contact surface side of the rear waistline region S2. The slit 54 is formed along the front-back direction in the center in the widthwise direction of the urination region. By the shrinkage of the rear leg hole elastic member 5R that crosses the absorber to the non-skin surface side thereof in the widthwise direction in the vicinity of the slit 54, the slit 54 functions as a curving unit that is formed such that the center in the widthwise direction of the absorber is bent convexly toward the wearer, and also functions as a guide portion that guides the body fluid from the urination region to the first region.

In the front leg hole elastic member 5F and the rear leg hole elastic member 5R of the disposable diaper 1D according to the fourth embodiment, the inside end of the leg hole elastic member in the widthwise direction extends to the center in the widthwise direction, and the right and left leg hole elastic members are continuous. The continuous leg hole elastic members are widthwise stretch members which are stretch in the widthwise direction.

Since the leg hole elastic member for the left leg and the leg hole elastic member for the right leg are arranged continuously, the leg hole elastic members can be lifted up together, thus allowing the rear waistline region to be lifted up efficiently. The rear leg hole elastic member is arranged between the slit formed in the absorber and the first region, and functions as a deformation restraining unit that restrains the deformation of the absorber in the urination region from being spread to the side of the first region R1 (the side of the rear waistline region).

It is noted that, the leg hole elastic member is desirably provided on the outer side in the front-back direction with respect to the slit formed in the absorber. For example, when the slit and the leg hole elastic member are arranged in an overlapping state, the absorber shrinks in the widthwise direction by the leg hole elastic member and the width of the slit is shortened in some cases. When the width of the slit is shortened, the function by the slit as a guide portion may be deteriorated. However, it is possible to realize both the function as a guide portion and the function as the deformation restraining unit by the separation between the leg hole elastic member and the slit.

In the above embodiment, the absorber was configured to bend from the slit and the compressed portion, however, the absorber may be configured to bend from a portion where the absorber is thin, or from a boundary portion where the rigidity changes in the absorber.

Further, the portion where the absorbent sheet and the first region are arranged may be to the topsheet side or to the exterior sheet side with respect to the absorber, or may be arranged inside the absorber. Further, the absorbent sheet may be arranged at the skin surface side of the wearer with respect to the topsheet. Further, the portion where the absorbent sheet and the first region are arranged is in the front waistline region S1.

In the present embodiment, the absorber 40 has a two-layered structure including the first layer 41 and the second layer 42 or a one-layered structure including the first layer only, however, the absorber 40 of the worn article according to the present invention can be configured by three or more layers.

An example is described where, as an example of the first region, the first region is formed by giving a higher basis weight of the absorbent polymer to one portion of the one-layered absorber, however, the first region may be formed such that the absorbent polymer may be arranged only in the central part in the widthwise direction of the absorber, or the absorbent polymer may be arranged over the entire width in the widthwise direction, or the absorbent polymer may be dispersed at the surface side of the absorber when seen in the cross-section of the absorber.

Thus, it is needless to say that the present invention includes various embodiments not specifically described herein. Therefore, the technical scope of the present invention is defined only by the inventive specific matter according to the scope of the claims appropriate from the above description.

[Reference Signs List]

| | |
|---|---|
| 1 | Disposable diaper (disposable wearing article); |
| 3 | Waist gathers; |
| 3A | Waist elastic member; |
| 4, 4' | Front waistband edge; |
| 5 | Leg gathers; |
| 5F | Front leg hole elastic member; |
| 5R | Rear leg hole elastic member; |
| 6, 6' | Rear waistband edge; |
| 7 | waistband gathers; |
| 7A | waistband elastic member; |
| 10 | Topsheet; |
| 15 | Second sheet; |
| 20 | Constriction portion; |
| 21 | Deformation restraining unit; |
| 30 | Absorber backside-covering sheet; |
| 40, 40G, 40H, 40J | Absorber; |
| 41 | First layer; |
| 41W | Inner end; |
| 42 | Second layer; |
| 42W | Outer end; |
| 43 | Elastic member-covering sheet; |
| 44 | Central elastic member; |
| 45 | Central aperture; |
| 46 | Side slit; |

| 49 | Side-end elastic member; |
|---|---|
| 50 | Leakage-preventing elastic member; |
| 53 | Compressed portion; |
| 53A | First compressed portion; |
| 53B | Second compressed portion; |
| 54 | Slit; |
| 54A | First slit; |
| 54B | Second slit; |
| 60 | Sidesheet; |
| 70F | Foreside exterior topsheet; |
| 70R | Rearside exterior topsheet; |
| 80F | Foreside exterior backsheet; |
| 80R | rearside exterior backsheet; |
| 90 | Absorbent sheet; |
| 92 and 93 | Liquid-permeable sheet; |
| 96 | absorbent polymer arranged region; |
| 96A | Inner end in front-back direction of absorbent polymer; |
| 97 | absorbent polymer non-arranged region; |
| 100 | Exterior center sheet; |
| 110 | Intermediate region; |
| R1 | First region; |
| R1A | Inner end in front-back direction of first region; |
| R2 | Second region; |
| S1 | Front waistline region; |
| S2 | Rear waistline region; |
| S3 | Urination region |

**Claims**

1. A disposable diaper, comprising:

   a liquid-permeable topsheet (10);
   a backsheet;
   an absorber (40G, 40H, 40J) arranged between the topsheet and the backsheet and having a front-back direction extending toward foreside and rearside of a wearer and a widthwise direction orthogonal to the front-back direction;
   an urination region (S3) which is a region in contact with an urination portion of the wearer;
   a buttocks region which is arranged to the rear of the urination region (S3) and is a region in contact with buttocks of the wearer;
   a ventral region which is arranged to the front of the urination region (S3) and is a region in contact with the ventral of the wearer, and
   exterior sheets which constitute the exterior portion of the disposable diaper (1),
   wherein a front waistband edge (4), positioned on the outside of the ventral region in a widthwise direction of the disposable diaper (1), is joined to a rear waistband edge (6), positioned on the outside of the buttocks region in the widthwise direction, whereby the disposable diaper (1) is formed into the pants-type,
   the absorber (40G, 40H, 40J) has a first region (R1) including absorbent polymer and a second region (R2) in which absorbent polymer has a lower basis weight than that in the first region (R1);
   the first region (R1) is only provided in the absorber of the ventral region; the second region (R2) is arranged in the absorber of the urination region (S3); and
   a deformation restraining unit (21) is formed to be capable of restraining deformation of the urination region (S3) from being propagated to the first region (R1) in the inner side in the front-back direction with respect to the inner end in the front-back direction of the first region (R1), wherein the deformation restraining unit (21) is configured by at least any one of: a constriction portion (20) that a side portion of the absorber is constricted inward in the widthwise direction; a slit formed in the absorber; a low basis weight region, in the absorber, that has a lower basis weight than that of the surrounding; a compressed portion (53) in which the absorber is compressed in a thickness direction; a widthwise stretch member that stretches in the widthwise direction; and a difference in rigidity of the absorber.

16

2. The disposable wearing article according to claim 1, wherein the deformation restraining unit (21) extends from the outer end of the absorber (40G, 40H, 40J) in the widthwise direction to the inner side in the widthwise direction.

3. The disposable wearing article according to any one of claims 1 to 2, wherein the deformation restraining unit (21) is formed to restrain the deformation of the urination region (S3) from being propagated to the buttocks region.

4. The disposable wearing article according to claim 3, wherein a guide portion that extends in the front-back direction and guides body fluid excreted to the urination region (S3) to the buttocks region, is formed in the absorber (40G, 40H, 40J) in the urination region (S3).

5. The disposable wearing article according to any one of claims 1 to 2, wherein the deformation restraining unit (21) is formed to restrain the deformation of the urination region (S3) from being propagated to the ventral region.

6. The disposable wearing article according to claim 5, wherein a guide portion that extends in the front-back direction and guides the body fluid excreted to the urination region (S3), to the ventral region, is formed in the absorber (40G, 40H, 40J) in the urination region (S3).

7. The disposable wearing article according to claim 4 or 6, wherein the guide portion is configured by at least one of: a slit that is formed in the absorber (40G, 40H, 40J); a low basis weight region in the absorber, that has a lower basis weight than that of the surrounding; and a compressed portion (53) in which the absorber is compressed in a thickness direction.

8. The disposable wearing article according to any one of claims 1 to 7, wherein a curving unit is formed such that the absorber (40G, 40H, 40J) is capable of bending convexly in the inner direction toward a wearer in the absorber (40G, 40H, 40J) of the urination region (S3).

**Patentansprüche**

1. Einwegwindel, umfassend:

   eine flüssigkeitsdurchlässige Oberschicht (10);
   eine Rückschicht;
   einen Absorber (40G, 40H, 40J), der zwischen der Oberschicht und der Rückschicht angeordnet ist und eine Vorwärts-Rückwärts-Richtung, die sich in Richtung der Vorderseite und Rückseite eines Trägers erstreckt, und eine Breitenrichtung orthogonal zur Vorwärts-Rückwärts-Richtung aufweist;
   einen Urinierbereich (S3), der ein Bereich ist, der mit einem Teil des Trägers zum Urinieren in Kontakt steht;
   einen Gesäßbereich, der hinter dem Urinierbereich (S3) angeordnet ist und ein Bereich ist, der mit dem Gesäß des Trägers in Kontakt steht;
   einen Bauchbereich, der vor dem Urinierbereich (S3) angeordnet ist und ein Bereich ist, der mit dem Bauch des Trägers in Kontakt steht, und
   Außenschichten, die den äußeren Abschnitt der Einwegwindel (1) bilden,
   wobei eine vordere Bundkante (4), die auf der Außenseite des Bauchbereichs in einer Breitenrichtung der Einwegwindel (1) positioniert ist, mit einer hinteren Bundkante (6) verbunden ist, die auf der Außenseite des Gesäßbereichs in Breitenrichtung positioniert ist, wodurch die Einwegwindel (1) zur Höschenart geformt ist,
   der Absorber (40G, 40H, 40J) einen ersten Bereich (R1), der absorbierendes Polymer beinhaltet, und einen zweiten Bereich (R2) aufweist, in dem absorbierendes Polymer ein geringeres Flächengewicht aufweist als das in dem ersten Bereich (R1);
   der erste Bereich (R1) nur im Absorber des Bauchbereichs bereitgestellt ist;
   der zweite Bereich (R2) in dem Absorber des Urinierbereichs (S3) angeordnet ist; und
   eine Verformungsbegrenzungseinheit (21) so ausgebildet ist, dass sie in der Lage ist, eine Verformung des Urinierbereichs (S3) so zu begrenzen, dass er sich auf den ersten Bereich (R1) auf der Innenseite in der Vorwärts-Rückwärts-Richtung in Bezug auf das innere Ende in der Vorwärts-Rückwärts-Richtung des ersten Bereichs (R1) ausbreitet, wobei die Verformungsbegrenzungseinheit (21) durch mindestens eines der Folgenden konfiguriert ist: einen Verengungsabschnitt (20), sodass ein Seitenabschnitt des Absorbers in der Breitenrichtung nach innen verengt ist; einen in dem Absorber ausgebildeten Schlitz; einen Bereich mit niedrigem Flächengewicht in dem Absorber, der ein geringeres Flächengewicht als das der Umgebung aufweist; einen komprimierten Abschnitt (53), in dem der Absorber in einer Dickenrichtung komprimiert ist; ein in der Breitenrichtung dehnbares Element, das sich in Breitenrichtung dehnt; und einen Unterschied in der Steifigkeit des Absorbers.

2. Einweg-Bekleidungsartikel nach Anspruch 1, wobei sich die Verformungsbegrenzungseinheit (21) vom äußeren Ende des Absorbers (40G, 40H, 40J) in Breitenrichtung zur Innenseite in Breitenrichtung erstreckt.

3. Einweg-Bekleidungsartikel nach einem der Ansprüche 1 bis 2, wobei die Verformungsbegrenzungseinheit (21) ausgebildet ist, um die Verformung des Urinierbereichs (S3) hinsichtlich der Ausbreitung in den Gesäßbereich zu begrenzen.

4. Einweg-Bekleidungsartikel nach Anspruch 3, wobei ein Führungsabschnitt, der sich in Vorderwärts-Rückwärts-Richtung erstreckt und die auf den Urinbereich (S3) ausgeschiedene Körperflüssigkeit in den Gesäßbereich führt, im Absorber (40G, 40H, 40J) im Urinierbereich (S3) ausgebildet ist.

5. Einweg-Bekleidungsartikel nach einem der Ansprüche 1 bis 2, wobei die Verformungsbegrenzungseinheit (21) ausgebildet ist, um die Verformung des Urinierbereichs (S3) dahingehend zu begrenzen, sich auf den Bauchbereich auszubreiten.

6. Einweg-Bekleidungsartikel nach Anspruch 5, wobei im Absorber (40G, 40H, 40J) im Urinierbereich (S3) ein Führungsabschnitt ausgebildet ist, der sich in Vorwärts-Rückwärts-Richtung erstreckt und die in den Urinierbereich (S3) ausgeschiedene Körperflüssigkeit in den Bauchbereich führt.

7. Einweg-Bekleidungsartikel nach Anspruch 4 oder 6, wobei der Führungsabschnitt durch mindestens eines der Folgenden konfiguriert ist: einen Schlitz, der in dem Absorber (40G, 40H, 40J) ausgebildet ist; einen Bereich mit niedrigem Flächengewicht in dem Absorber, der ein geringeres Flächengewicht als das der Umgebung aufweist; und einen komprimierten Abschnitt (53), in dem der Absorber in einer Dickenrichtung komprimiert ist.

8. Einweg-Bekleidungsartikel nach einem der Ansprüche 1 bis 7, wobei eine Krümmungseinheit so ausgebildet ist, dass der Absorber (40G, 40H, 40J) in der Lage ist, sich konvex in der Innenrichtung zu einem Träger im Absorber (40G, 40H, 40J) des Urinierbereichs (S3) zu biegen.


**Revendications**

1. Couche jetable, comprenant :

    une feuille supérieure perméable aux liquides (10) ;
    une feuille inférieure ;
    un absorbeur (40G, 40H, 40J) agencé entre la feuille supérieure et la feuille inférieure et ayant une direction avant-arrière s'étendant vers le côté avant et le côté arrière d'un utilisateur et une direction dans le sens de la largeur orthogonale à la direction avant-arrière ;
    une région de miction (S3) qui est une région en contact avec une partie de miction de l'utilisateur ;
    une région fesses qui est agencée à l'arrière de la région de miction (S3) et est une région en contact avec les fesses de l'utilisateur ;
    une région ventrale qui est agencée à l'avant de la région de miction (S3) et est une région en contact avec le ventre de l'utilisateur, et
    des feuilles extérieures qui constituent la partie extérieure de la couche jetable (1),
    dans laquelle un bord de ceinture avant (4), positionné sur l'extérieur de la région ventrale dans une direction dans le sens de la largeur de la couche jetable (1), est lié à un bord de ceinture arrière (6), positionné sur l'extérieur de la région fesses dans la direction dans le sens de la largeur, moyennant quoi la couche jetable (1) est formée selon le type culotte,
    l'absorbeur (40G, 40H, 40J) comporte une première région (R1) incluant un polymère absorbant et une seconde région (R2) dans laquelle un polymère absorbant a un poids de base inférieur à celui dans la première région (R1) ;
    la première région (R1) se situe seulement dans l'absorbeur de la région ventrale ;
    la seconde région (R2) est agencée dans l'absorbeur de la région de miction (S3) ; et
    une unité de limitation de déformation (21) est formée pour être capable de limiter la propagation de la déformation de la région de miction (S3) vers la première région (R1) dans le côté intérieur dans la direction avant-arrière par rapport à l'extrémité intérieure dans la direction avant-arrière de la première région (R1), dans laquelle l'unité de limitation de déformation (21) est configurée par au moins l'un parmi : une partie de constriction (20) qui fait qu'une partie latérale de l'absorbeur est contrainte vers l'intérieur dans la direction dans le sens de la largeur ; une fente formée dans l'absorbeur ; une région de poids de base faible, dans l'absorbeur, qui a un poids de base inférieur à celui des environs ; une partie comprimée (53) dans laquelle l'absorbeur est comprimé dans une direction d'épaisseur ; un élément extensible dans le sens de la largeur qui s'étire dans la direction dans le sens de la largeur ; et une différence de rigidité de l'absorbeur.

**2.** Article jetable à porter selon la revendication 1, dans lequel l'unité de limitation de déformation (21) s'étend depuis l'extrémité extérieure de l'absorbeur (40G, 40H, 40J) dans la direction dans le sens de la largeur vers le côté intérieur dans la direction dans le sens de la largeur.

**3.** Article jetable à porter selon l'une quelconque des revendications 1 à 2, dans lequel l'unité de limitation de déformation (21) est formée pour limiter la propagation de la déformation de la région de miction (S3) vers la région fesses.

**4.** Article jetable à porter selon la revendication 3, dans lequel une partie de guidage qui s'étend dans la direction avant-arrière et guide le fluide corporel excrété vers la région de miction (S3) jusqu'à la région fesses, est formée dans l'absorbeur (40G, 40H, 40J) dans la région de miction (S3).

**5.** Article jetable à porter selon l'une quelconque des revendications 1 à 2, dans lequel l'unité de limitation de déformation (21) est formée pour limiter la propagation de la déformation de la région de miction (S3) vers la région ventrale.

**6.** Article jetable à porter selon la revendication 5, dans lequel une partie de guidage qui s'étend dans la direction avant-arrière et guide le fluide corporel excrété vers la région de miction (S3), jusqu'à la région ventrale, est formée dans l'absorbeur (40G, 40H, 40J) dans la région de miction (S3).

**7.** Article jetable à porter selon la revendication 4 ou 6, dans lequel la partie de guidage est configurée par au moins l'une parmi : une fente qui est formée dans l'absorbeur (40G, 40H, 40J) ; une région de poids de base faible dans l'absorbeur, qui a un poids de base inférieur à celui des environs ; et une partie comprimée (53) dans laquelle l'absorbeur est comprimé dans une direction d'épaisseur.

**8.** Article jetable à porter selon l'une quelconque des revendications 1 à 7, dans lequel une unité d'incurvation est formée de telle sorte que l'absorbeur (40G, 40H, 40J) est capable de se plier de manière convexe dans la direction intérieure vers un utilisateur dans l'absorbeur (40G, 40H, 40J) de la région de miction (S3).

# FIG. 1

# FIG. 2

FIG. 3

FIG. 4

FIG. 5

# FIG. 6

# FIG. 7

# FIG. 8

# FIG. 9

# FIG. 10

FIG. 11

# FIG. 12

FIG. 13

# FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0866426 B

- JP 2006346439 A